# EUROPÄISCHE PATENTSCHRIFT

(11) **EP 0 715 832 B1**
(45) Veröffentlichungstag und Bekanntmachung des Hinweises auf die Patenterteilung: **02.01.2002**
(21) Anmeldenummer: 95116324.5
(22) Anmeldetag: 17.10.1995
(51) Int. Cl.: A61B 17/58

(54) **Marknagel zur Hüftkompression**
Intramedullary nail for hip compression
Broche intramédullaire pour compression de la hanche

(30) Priorität: 17.11.1994 DE 4440797; 17.11.1994 DE 4440799
(43) Veröffentlichungstag der Anmeldung: 12.06.1996
(73) Patentinhaber: Treu-Instrumente GmbH, D-78532 Tuttlingen (DE)
(72) Erfinder: Treu, Ernst, 78532 Tuttlingen (DE); Kupferschmid, Thomas, 78194 Immendingen (DE); Tawadrous Mouris Fouad Dr. c/o Americo Medical, Giza-Cairo (EG)
(74) Vertreter: Hiebsch, Gerhard F., Dipl.-Ing.

(56) Entgegenhaltungen:
- EP-A- 0 337 288
- EP-A- 0 355 411
- EP-A- 0 514 662
- WO-A-93/11713
- DE-A- 3 244 243
- DE-U- 29 504 111
- US-A- 4 622 959
- US-A- 5 167 663

## Beschreibung

Die Erfindung betrifft einen Marknagel mit zwei im Bereich eines -- an seinen Nagelschaft anschließenden -- Kupplungsabschnittes seine Längsachse querenden und zu ihr in einem Neigungswinkel verlaufenden Bohrungen zum Führen einer Schaftschraube oder Zugschraube zur Hüftkompression.

Marknägel mit einer endwärtigen Spitze werden in der Operationstechnik zur Nagelung von Knochenbrüchen eingesetzt. Beispielsweise setzt der Operateur bei einem Femurbruch in der Schaftachse eine Ahle an oder einen Führungsdraht in der Mitte der Fossa trochantrica. Eine Kugelspitze des Führungsdrahtes wird bis zur Frakturstelle eingeschoben, dann wird bei einer der Operationsmethoden der so entstandene Kanal zur Einführung des Marknagels aufgefräst und letzterer eingeschlagen. Anschließend erfolgt eine distale Verriegelung mittels wenigstens einer den Marknagel durchsetzenden Schraube, deren beide Enden im Knochen festliegen. Selbstverständlich kann ein Nagel auch ohne Auffräsung eingebracht werden.

Es werden sog. Verriegelungsschrauben eingesetzt, beispielsweise bei einer Tibia zumindest zwei Verriegelungsschrauben. Diese werden zur Extraktion des Nagels entfernt, ein Ausschlägeradapter auf das proximale Ende des Marknagels geschraubt und letzterer mit einem Schlitzhammer ausgetrieben.

Marknägel sind entweder -- bevorzugt anterior -- der Länge nach mit einem Schlitz versehen oder weisen einen geschlossenen Querschnitt von beispielsweise 8 mm bis 16 mm Durchmesser auf, die Verriegelungsschrauben haben ein Teilgewinde mit einem Außendurchmesser.

Einen Marknagel der eingangs erwähnten Form offenbart die EP 0 355 411, dessen Querschnitt seitliche Nuteinformungen und einen Innenkanal erkennen lässt. Der Einsatz dieses Marknagels ist auf Standardfrakturen im Femur beschränkt; eine Versorgung für isolierte mediale Schenkelhalsfrakturen (Kontraindikation) ist nicht zu gewährleisten, da eine retrograde Verriegelung unmöglich bleibt.

Ein Knochennagel für lange Röhrenknochen besteht nach DE 32 44 243 A1 aus einem röhrenförmigen Grundkörper, der distal mit einer Spitze versehen ist und proximal einen Kopf zum Ansetzen eines Einschlaginstrumentes aufweist. Der distale Randbereich des Grundkörpers weist einen geschlossenen Querschnitt mit zwei Lochpaaren auf; die beiden Löcher eines Lochpaares liegen koaxial auf einer gemeinsamen, den Nagel quer zu seiner Längsrichtung durchsetzenden Achse und die beiden Achsen der beiden Lochpaare in verschiedenen, quer zur Längsrichtung des Nagels verlaufenden Ebene. Diese Achsen kreuzen sich unter einem spitzen Winkel von vorzugsweise zwischen 10° und 60°. Der an den distalen Randbereich anschließende Mittelbereich ist in Längsrichtung des Nagels geschlitzt, wobei der Schlitz durch Verbiegen seiner Ränder erweitert ist; dieser Mittelbereich weist einen Querschnitt auf, der größer ist als der geschlossene Querschnitt des distalen Randbereiches. Um einen leicht einzuschlagenden Knochennagel zu schaffen, der zudem eine sichere Fixierung der Bruchstelle bewirkt und sowohl für rechte als auch für linke Knochen eingesetzt zu werden vermag, soll der an den Mittelbereich anschließende proximale Randbereich wieder einen geschlossenen, dem distalen Randbereich entsprechenden Querschnitt aufweisen und mit zwei als Gewindebohrungen gestalteten Lochpaaren versehen sein, die mit ihren Achsen in einer gemeinsamen Ebene liegen. Letztere verläuft zur Ebene, in der die gekrümmte Nagelachse liegt, im wesentlichen senkrecht und in Richtung der Nagelachse im Bereich der besagten Lochpaare, die sich im Nagelinneren -- bevorzugt etwa rechtwinkelig -- schneiden.

Die US-PS 5,167,663 beschreibt einen Marknagel mit Längsrinnen sowie mit einem Paar parallel geneigter Bohrungen in jenem gegenüber dem Hauptteil des Marknagels querschnittlich erweiterten Kupplungsabschnitt. Zudem wird ein Zielbügel beschrieben, der an Mitnehmer der Endkante des Kupplungsabschnittes durch entsprechende Gegenelemente lösbar angeschlossen wird und in jenem Neigungswinkel parallele Führungskanäle für die Schaftschrauben anbietet. Um der sog. Anteversion des Hüfthalses -- einer Vorwärtsneigung -- von 8° zu begegnen, wird der Marknagel um jene 8° verdreht in den Markraumkanal eingetrieben.

Diese Literaturstelle offenbart ein Paar paralleler Bohrungen deshalb, weil in pertrochantärer Stellung zwei Schaftschrauben bevorzugt werden, hingegen subtrochantär deren eine möglich bleibt.

Da es zum einen schwierig ist, auf diese Weise den Anteversionswinkel einzustellen -- der Marknagel folgt beim Einschlagen der Markraumröhre -- und man zum anderen linke und rechte Marknägel mit der Folge aufwendiger Lagerhaltung benötigt, hat sich der Erfinder das Ziel gesetzt, einen Marknagel der eingangs erwähnten Art so auszugestalten, daß die erwähnten Mängel nicht auftreten und eine vielseitige Verwendung möglich wird.

Zur Lösung dieser Aufgabe führt die Lehre der unabhängigen Patentansprüche; die Unteransprüche geben günstige Weiterbildungen an.

Erfindungsgemäß weist der Marknagel jeweils in einem Neigungswinkel -- mit entgegengesetzten Neigungen -- verlaufende Bohrungen zum Führen der Zug- oder Schaftschraube zur Hüftkompression auf, wobei für einen Einsatz als rechter oder linker Marknagel die Mündungen der Bohrungen mit einem Umfangsabstand zueinander -- der kürzer als ihr Durchmesser ist -- angebracht sind sowie die Mittelachsen der beiden Bohrungen mit einer zwischen ihnen durch die Längsachse des Marknagels gelegten Bezugsebene jeweils einen Querschnittswinkel von etwa 8° begrenzen. Im Rahmen dieser Erfindung ist insbesondere für pertrochantären Einsatz jeder der beiden Bohrungen in axialem Abstand eine parallele Bohrung für eine weitere Schaftschraube gleicher Befestiungsrichtung zugeordnet.

Von Vorteil ist auch, die erwähnte Bezugsebene als Symmetrieebene des Nagelschaftes heranzuziehen.

Dank dieser Maßgabe entsteht ein Marknagel, der sowohl für rechte als auch für linke Hüften eingesetzt werden kann und das durch die Anteversion des Hüfthalses entstehenden Probleme durch die besondere Lage der Bohrungspaare zueinander zu kompensieren vermag.

Nach einem weiteren Merkmal der Erfindung bilden die Mittelachsen der beiden Bohrungen bzw. Bohrungspaare querschnittlich einen Winkel von 16°. Zudem hat es sich als günstig erwiesen, zwischen den Bohrungen eines parallelen Bohrungspaares eine Bohrung des anderen Bohrungspaares verlaufen zu lassen, d. h. die Bohrungspaare liegen dichtestmöglich aneinander.

Zur besseren Führung des Marknagels sollen noch in seine Umfangsfläche auf dieser verteilte Längsrinnen eingeformt sein. Außerdem ist der Marknagel im Bereich der Endkante seines Kupplungsabschnitts mit wenigstens zwei etwa achsparallelen Eingriffschlitzen sowie einem Innengewinde für ein Gegengewinde eines Adapterbolzens zur Verbindung des Mark-Erfindungsgemäß weist der Marknagel jeweils in einem Neigungswinkel -- mit entgegengesetzten Neigungen -- verlaufende Bohrungen zum Führen der Zug- oder Schaftschraube zur Hüftkompression auf, sind für einen Einsatz als rechter oder linker Marknagel die Mündungen der Bohrungen mit einem Umfangsabstand zueinander, der kürzer als ihr Durchmesser ist, angebracht, und die Mittelachsen der beiden Bohrungen begrenzen mit einer zwischen ihnen durch die Längsachse des Marknagels gelegten Bezugsebene jeweils einen Querschnittswinkel von etwa 8°. Im Rahmen dieser Erfindung ist insbesondere für pertrochantären Einsatz jeder der beiden Bohrungen in axialem Abstand eine parallele Bohrung für eine weitere Schaftschraube gleicher Befestiungsrichtung zugeordnet.

Von Vorteil ist auch, die erwähnte Bezugsebene als Symmetrieebene des Nagelschaftes heranzuziehen.

Dank dieser Maßgabe entsteht ein Marknagel, der sowohl für rechte als äuch für linke Hüften eingesetzt werden kann und das durch die Anteversion des Hüfthalses entstehenden Probleme durch die besondere Lage der Bohrungspaare zueinander zu kompensieren vermag.

Nach einem weiteren Merkmal der Erfindung bilden die Mittelachsen der beiden Bohrungen bzw. Bohrungspaare querschnittlich einen Winkel von 16°. Zudem hat es sich als günstig erwiesen, zwischen den Bohrungen eines parallelen Bohrungspaares eine Bohrung des anderen Bohrungspaares verlaufen zu lassen, d. h. die Bohrungspaare liegen dichtestmöglich aneinander.

Zur besseren Führung des Marknagels sollen noch in seine Umfangsfläche auf dieser verteilte Längsrinnen eingeformt sein. Außerdem ist der Marknagel im Bereich der Endkante seines Kupplungsabschnitts mit wenigstens zwei etwa achsparallelen Eingriffschlitzen sowie einem Innengewinde für ein Gegengewinde eines Adapterbolzens zur Verbindung des Marknagels mit einem Zielbügel ausgestattet; eine vom Adapterbolzen durchsetzte Führungshülse eines Zielbügels ist mit Gegenelementen für den Eingriffschlitz des Marknagels versehen, die entweder mit der/den linksgerichteten Bohrung/en oder mit der/den rechtsgerichteten Bohrung/en haltend zusamenwirken.

Der erfindungsgemäße Marknagel wird durch zwei in den Hüfthals ragenden und parallel verlaufenden selbstschneidenden -- mit Längsrinnen oder Kanneluren ausgestatteten -- Schaftschrauben ergänzt; man kann folglich zwei Bohrdrähte über den Zielbügel durch eine Bohrhülse einbringen. Dabei wird die Hüftkugel gesichert, und es werden beim Eindrehen der Schaftschrauben keine unerwünschte Rotation stattfinden. Eine solche Rotation ist beispielsweise bei einem System nach US-PS 5,167,663 ohne weiteres möglich. Findet aber bei einem Hüfthalsbruch oder Hüftkapselbruch eine Rotation statt, wird die obere Blutzufuhr unterbrochen, der Knochen ist tot. Als letzte Möglichkeit einer Behandlung verbleibt dann, den Hüfthals abzusägen und eine Hüftprothese einzusetzen.

Am distalen Ende des Marknagels werden zwei Kanneluren aufweisende Verriegelungsbolzen mit Vollgewinde -- also selbstschneidend -- vorgesehen, die ebenfalls über Führungsdrähte eingedreht werden. Hierdurch wird ein sicheres Eindrehen gewährleistet.

Weitere Vorteile, Merkmale und Einzelheiten der Erfindung ergeben sich aus der nachfolgenden Beschreibung bevorzugter Ausführungsbeispiele sowie anhand der Zeichnung; diese zeigt in
- Fig. 1:: eine Seitenansicht eines Oberschenkelknochens mit Hüftkopf sowie einem Zielgerät zum Einbringen von Bohrdrähten;
- Fig. 2:: einen schematisierten Längsschnitt durch den Oberschenkelknochen mit darin festgelegtem Marknagel zur Hüftkompression;
- Fig. 3:: eine Seitenansicht des Marknagels;
- Fig. 4:: einen Längsschnitt durch den Marknagel der Fig. 3 in deren Linie A;
- Fig. 5:: einen vergrößerten Teillängsschnitt durch die Nagelspitze gemäß Pfeil V in Fig. 3;
- Fig. 6, 7:: vergrößerte Querschnitte durch den Marknagel nach den Schnittlinien VI-VI bzw. VII-VII der Fig. 3;
- Fig. 8:: eine teilweise geschnittene Seitenansicht eines Zielbügels mit Führungshülse;
- Fig. 9:: die Frontansicht der Führungshülse;
- Fig. 10:: die Seitenansicht eines Zusatzteils zum Zielbügel;
- Fig. 11:: eine teilweise geschnittene Seitenansicht eines Adapterbolzens;
- Fig. 12:: die Frontansicht des Adapterbolzens;

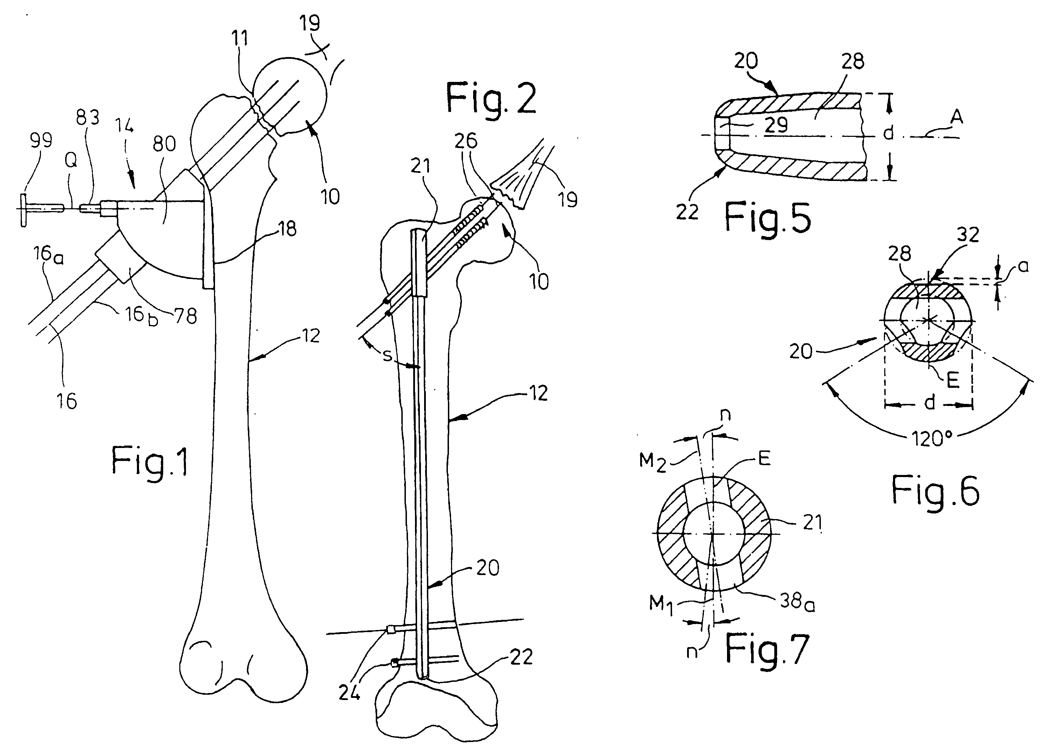

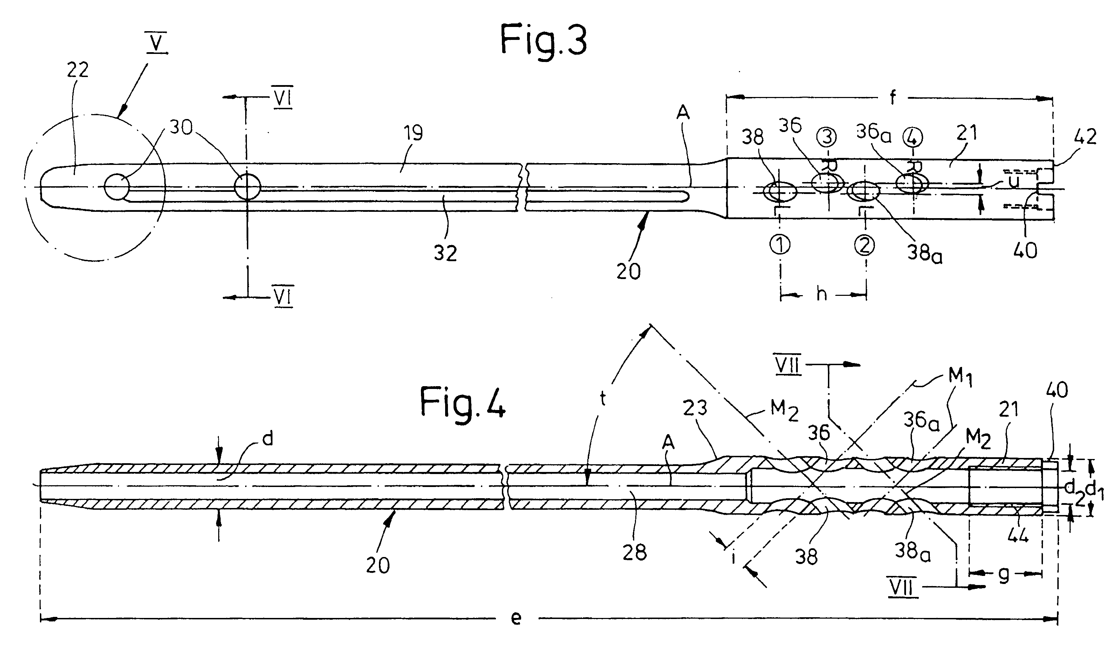

An einem Hüftkopf 10 eines bei 12 angedeuteten Oberschenkelknochens ist in Fig. 1 ein Zielgerät 14 zum Einschießen dreier Bohrdrähte 16, 16ₐ, 16_{b} -- (Durchmesser: 2,5 mm) -- zu erkennen, das mit einer konkaven Fläche 18 jenem Oberschenkelknochen 12 anliegt.

Der mittlere Bohrdraht 16 dient zum Einsetzen einer in Fig. 1 nicht dargestellten Schaftschraube, die flankierenden Bohrdrähte 16ₐ, 16_{b} sollen den bei einem Bruch 11 abgetrennten Hüftkopf 10 gegen Verdrehen beim Einbringen jener Zug- oder Schaftschraube sichern; bei einem Schenkelhalsbruch und der Unterbrechung der Blutzufuhr -- Bereich 19 -- am Bruch 11 wird ansonsten eine Rotation möglich.

Fig. 2 verdeutlicht im Oberschenkelknochen 12 einen Marknagel 20, dessen distales Ende 22 -- zusammen mit dem Oberschenkelknochen 12 -- von zwei Verriegelungsbolzen 24 durchsetzt ist. Ein an einen Nagelschaft 19 anchließender proximaler Kupplungsabschnitt 21 des Marknagels 20 nimmt zu dessen Längsachse A in einem Neigungswinkel w verlaufende Schaftschrauben 26 auf, welche die Hüftkugel bzw. den Hüftkopf 10 an die Bruchfläche ziehen.

Der Marknagel 20 der Fig. 3 bis 7 ist hohl, und sein Hohlraum 28 an dem sich geringfügig konisch verjüngenden distalen Ende 22 offen (Bohrung 29). Der Außendurchmesser d mißt bei einem für einen unaufgebohrten Markraum bestimmten Marknagel 20 beispielsweise 9 mm, die Länge e zwischen 240 mm und 380 mm. Diesen Maßen entsprechen bei einem Marknagel 20 für einen aufgebohrten Markraum beispielshaft d = 13 mm und e = 320 mm bis 440 mm.

In Fig. 3 sind zwei Querbohrungen 30 für jene Verriegelungsbolzen 24 zu erkennen, wobei von der dem distalen Ende 22 näheren drei -- gleichmäßig am Umfang des Nagelschaftes 19 verteilte -- kannelurenartige Längsrinnen 32 der Tiefe a von 0,5 mm ausgehen, die am Kupplungsabschnitt 21 der Länge f von hier 75 mm enden; an dessen gerundeten Übergang 23 erweitert sich der Außendurchmesser d₁ auf 13 mm bei dem erwähnten Beispiel eines Marknagels 20 für den unaufgebohrten Markraum bzw. auf 15 mm im zweitgenannten Fall. Im übrigen sind auch die Verriegelungsbolzen 24 mit Kanneluren sowie mit einem selbstschneidenen Vollgewinde ausgestattet.

Der Kupplungsabschnitt 21 des Marknagels 20 enthält zwei gegenläufige Paare von jeweils in einem Abstand h von 19 mm parallelen Bohrungen 36, 36ₐ und 38, 38ₐ. Deren Durchmesser i mißt 5,3 mm bzw. 6,8 mm, und deren Achsen M₁ bzw. M₂ schließen mit der Längsachse A des Marknagels 20 einen Winkel t von 45° ein. Nach Fig. 3,4 liegt zwischen zwei parallelen Bohrungen 36,36ₐ oder 38,38ₐ jeweils eine Bohrung 38ₐ oder 36ₐ des anderen Bohrungspaares.

In Fig. 3 sind Gebrauchsmarkierungen R (für rechts) und L (für links) sowie Bohrungsbezifferungen "1" bis "4" für den Operateur zu erkennen. Zudem ist mit 40 ein Eingriffschlitz an der Endkante 42 des Kupplungsabschnitts 21 bezeichnet, an die ein Kupplungs- oder Innengewinde 44 der Länge g von 16,4 mm mit einem Innendurchmesser d₂ von 8,5 mm anschließt.

Wie besonders Fig. 7 zu veranschaulichen trachtet, bestimmen die Achsenpaare M₁ und M₂ mit einer zwischen ihnen verlaufenden Bezugsebene E durch jene Längsachse A in einem Bezugsdurchmesser des Querschnitts jeweils einen Querschnittswinkel n von 8°; der in Fig. 3 erkennbare Umfangsabstand u der Mündungen zweier benachbarter -- gegenläufiger -- Bohrungen 36, 38 beträgt hier etwa 2,6 mm.

Diese Anordnung der Paare von Bohrungen 36, 36ₐ bzw. 38, 38ₐ erlaubt es -- trotz der sog. Anteversion der Hüfthälse, also deren Vorwärtsneigung -- mit einer einzigen Form des Marknagels 20 für beide Oberschenkelknochen 12 eines Menschen auszukommen; der Lagerhaltung je einer Nagelserie für die rechte und für die linke Körperhälfte bedarf es so nicht mehr.

Um die -- ihrerseits ebenfalls selbstschneidenden -- Schaftschraube/n 26 einbringen zu können, wird jener Endkante 42 des Kupplungsabschnitts 21 proximal ein Zielbügel 46 aufgesetzt. Dieser ist ein Winkelstück zum einen mit einem kurzen Arm 47 für eine Führungshülse 48 für den Marknagel 20 sowie zum anderen mit einem längeren Arm 49 der Länge q von 180 mm, in dem im Neigungswinkel w zur Längsachse A geneigte Führungskanäle 50 für die Schaftschrauben 26 verlaufen. Bei 52 ist die Lage der Bohrung 36,38 des der Führungshülse 48 zugeordneten -- hier nicht gezeigten -- Marknagels 20 angedeutet. In eine Sackbohrung 52 des langen Arms 49 wird das Schraubenende 54 eine Handgriffes 56 eingesetzt.

Für den beschriebenen Marknagel 20 sind zwei derartige Zielbügel 46 mit unterschiedlichen Lagen der fixierten Führungshülse 48 erforderlich; gemäß Fig. 9 sind Gegenelemente 58 für den Eingriffschlitz 40 des Marknagels 20 so angebracht, daß sie in einem Anwendungsfall mit den linksgerichteten Bohrungen 36,36ₐ und im anderen Falle mit den dazu gewinkelten Bohrungen 38,38a zusammenwirken. Die beiden in Fig. 9 gezeigten Winkel n entsprechen jenen in Fig. 7.

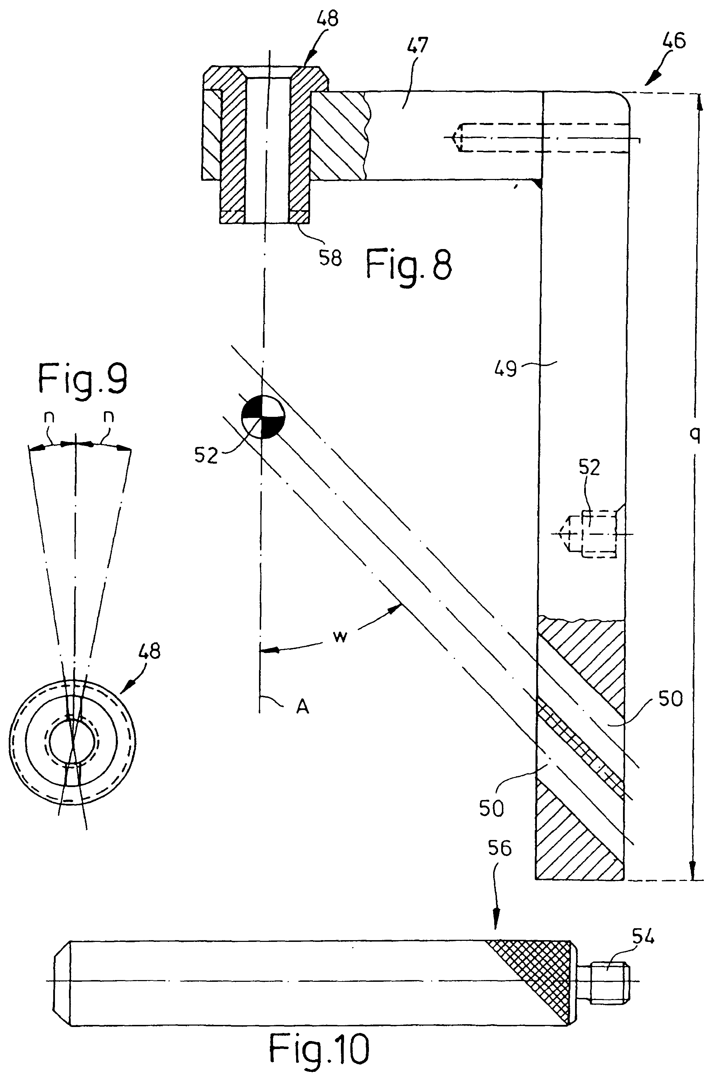

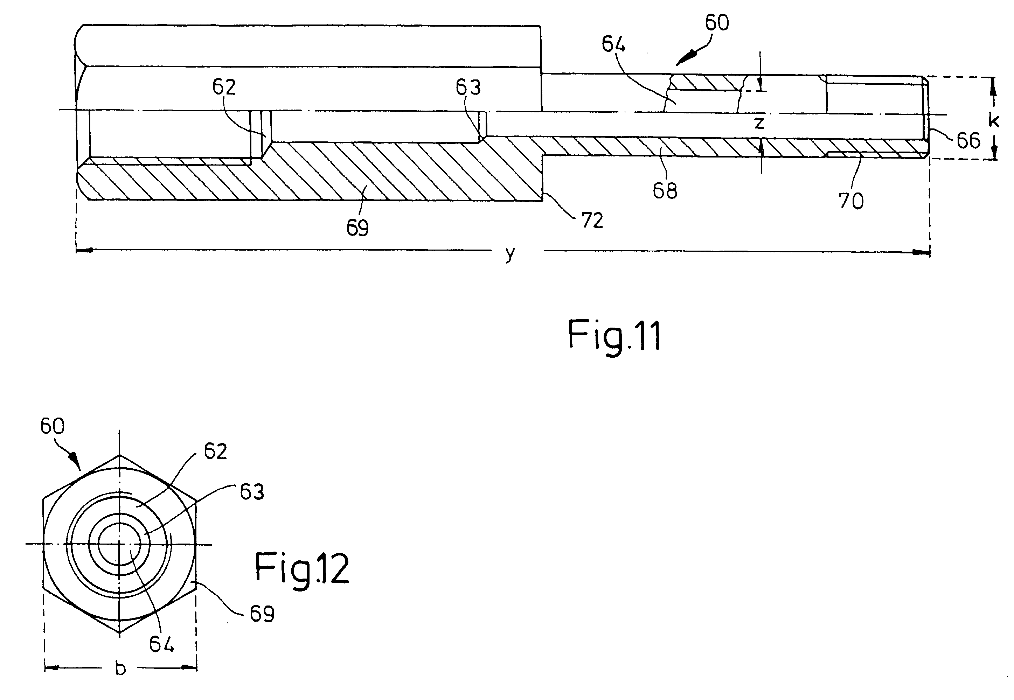

Die Fig. 11,12 geben einen gegenüber dem Zielbügel 46 der Fig. 8 vergrößerten Adapterbolzen 60 der Länge y von 100 mm mit längsschnittlich bei 62,63 zweifach gestuftem Innenraum 64 der engsten Weite z von etwa 5 mm wieder; dieser wird mit der freien Kante 66 eines querschnittlich runden Bolzenendes 68 mit Außengewinde 70 -- dessen Außendurchmesser k dem Innendurchmesser d₂ des Kupplungsgewindes 44 entspricht -- in die Führungshülse 48 eingeschoben und im Marknagel 20 festgeschraubt.

Die Stufenschulter zwischen dem runden Bolzenende 68 und dem polygonen Anschlußstück 69 der maximalen Breite b von 17 mm des Adapterbolzens 60 ist in Fig. 7 mit 72 bezeichnet.

Das Zielgerät 14 liegt in der in Fig. 1 skizzierten Arbeitsstellung mit seiner rechtwinkeligen und querschnittlich konkaven Formfläche 18 eines Profilstreifens 74 dem Oberschenkelknochen 12 nahe dem Hüftkopf 10a. Der Profilstreifen 74 schließt an einen eine schwenkbar angelenkte Zielplatte 78 aufnehmenden hohlen Haltekörper 80 an. Diese Zielplatte 78 enthält drei oder mehr Drahtführungskanäle.

Der Neigungswinkel der Längsachse des mittleren Drahtführungskanals zu einer durch fluchtende Laschenaugen gelegten Parallelen zu einer Bezugsachse Q des Zielgerätes 14, die durch enen Griffanschluß 83 gelegt sei, ist mittels Rasten einer Zahnung im Haltekörper 80 festlegbar und zwar bevorzugt bei Winkel von 45°, 50°, 55°, 60°, was Arbeitswinkeln des Zielgerätes 14 in Arbeitsstellung von 135°, 140°, 145° und 150° entspricht.

Das -- wie gesagt -- mit seiner konkaven Formfläche 18 dem Oberschenkelknochen 12 in Arbeitsstellung angelegte Zielgerät 14 wird an diesem einerseits durch spitze Einschubnoppen sowie anderseits gemäß Fig. 1 durch einen Griff 99 gehalten. Dann werden die Bohrdrähte 16,16ₐ,16_{b} mittels eines nicht gezeigten Bohrgerätes drehend eingetrieben.

## Patentansprüche

1. Marknagel mit zwei im Bereich eines an seinem Nagelschaft anschließenden, proximalen Kupplungsabschnittes (21) seine Längsachse (A) querenden und zu ihr in einem Neigungswinkel (t) verlaufenden Bohrungen (36, 38) zum Führen einer Zuq- oder Schaftschraube (26) zur Hüftkompression, **dadurch gekennzeichnet, daß** für einen Einsatz als rechter oder linker Marknagel (20) die Mündungen der Bohrungen mit einem Umfangsabstand (u) zueinander, der kürzer als ihr Durchmesser (i) ist, angebracht sind, wobei die Mittelachsen (M₁ bzw. M₂) der beiden Bohrungen mit einer zwischen ihnen durch die Längsachse (A) des Marknagels (20) gelegten Bezugsebene (E) jeweils einen Querschnittswinkel (n) von etwa 8° begrenzen.

2. Marknagel nach Anspruch 1, **dadurch gekennzeichnet, dass** jeder der beiden Bohrungen (36, 38) in axialem Abstand (h) eine parallele Bohrung (36ₐ, 38ₐ) für eine weitere Schaftschraube (26) zugeordnet ist.

3. Marknagel nach Anspruch 1 oder 2, **dadurch gekennzeichnet, daß** die Mittelachsen (M₁, M₂) der beiden Bohrungen (36 und 38) bzw. Bohrungspaare (36, 36ₐ und 38, 38ₐ) querschnittlich einen Winkel von 16° bilden.

4. Marknagel nach Anspruch 2 oder 3, **dadurch gekennzeichnet, dass** zwischen den Bohrungen (36, 36ₐ bzw. 38, 38ₐ) eines parallelen Bohrungspaares eine Bohrung (38ₐ bzw. 36) des anderen Bohrungspaares verläuft.

5. Marknagel nach einem der Ansprüche 1 bis 4, **dadurch gekennzeichnet, daß** die Bezugsebene (E) Symmetrieebene des an den Kupplungsabschnitt (21) anschließenden Nagelschaftes (19) ist.

6. Marknagel nach einem der Ansprüche 1 bis 5, **dadurch gekennzeichnet, daß** der Kupplungsabschnitt (21) im Bereich seiner Endkante (42) wenigstens zwei etwa achsparallelen Eingriffschlitze (40) sowie ein Innengewinde (44) für ein Gegengewinde (70) eines Adapterbolzens (60) zur Verbindung des Marknagels (20) mit einem Zielbügel (46) aufweist, wobei eine vom Adapterbolzen (60) durchsetzte Führungshülse (48) eines Zielbügels (46) mit Gegenelementen (58) für den Eingriffschlitz (40) des Marknagels (20) versehen ist, die entweder mit der/den linksgerichteten Bohrung/en (36, 36ₐ) oder mit der/den rechts gerichteten Bohrung/en (38, 38ₐ) zusammenwirken.

7. Marknagel nach einem der Ansprüche 1 bis 6, **dadurch gekennzeichnet, daß** die Schaftschraube/n (26) Längsrinnen sowie ein selbstschneidendes Gewinde aufweist/aufweisen.

8. Marknagel mit Querbohrungen für Verriegelungsschrauben nach einem der Ansprüche 1 bis 7, **dadurch gekennzeichnet, daß** die Verriegelungsschraube/n (24) Längsrinnen sowie ein selbstschneidendes Gewinde aufweist/aufweisen.

## Claims

1. Intramedullary nail with two bores (36, 38) traversing its longitudinal axis (A) in the region of a proximal coupling section (21) connected to its nail shank and extending at an angle of inclination (t) thereto for guiding a compression screw or shank screw (26) for hip compression, **characterised in that** the mouths of the bores are disposed at a circumferential distance (u) from one another which is shorter than their diameter (i) for use as right or left intramedullary nails (20), the centre axes (M₁ and M₂) of the two bores each delimiting a cross-sectional angle (n) of approximately 8° together with a reference plane (E) situated between them through the longitudinal axis (A) of the intramedullary nail (20).

2. Intramedullary nail according to claim 1, **characterised in that** each of the two bores (36, 38) has an associated parallel bore (36ₐ, 38ₐ) for another set screw (26) at an axial distance (h) therefrom.

3. Intramedullary nail according to claim 1 or claim 2, **characterised in that** the centre axes (M₁, M₂) of the two bores (36 and 38) or bore pairs (36, 36ₐ and 38, 38ₐ) form a cross-sectional angle of 16°.

4. Intramedullary nail according to claim 2 or claim 3, **characterised in that** between the bores (36, 36ₐ and 38, 38ₐ) of a parallel bore pair extends a bore (38ₐ or 36) of the other bore pair.

5. Intramedullary nail according to one of claims 1 to 4, **characterised in that** the reference plane (E) is a plane of symmetry of the nail shank (19) connected to the coupling section (21).

6. Intramedullary nail according to one of claims 1 to 5, **characterised in that** the coupling section (21) is provided in the region of its end edge (42) with at least two substantially axially parallel engagement slots (40) and an internal thread (44) for a mating thread (70) of an adapter bolt (60) for connecting the intramedullary nail (20) to an aligning bracket (46), a guide sleeve (48) of an aligning bracket (46) traversed by the adapter bolt (60) being provided with mating elements (58) for the engagement slot (40) of the intramedullary nail (20), cooperating either with the left-hand bore(s) (36, 36ₐ) or with the right-hand bore(s) (38, 38ₐ),

7. Intramedullary nail according to one of claims 1 to 6, **characterised in that** the set screw(s) (26) has/have longitudinal channels and a self-cutting thread.

8. Intramedullary nail with transverse bores for interlocking screws according to one of claims 1 to 7, **characterised in that** the interlocking screw(s) (24) has/have longitudinal channels and a self-cutting thread.

## Revendications

1. Broche d'ostéosynthèse intra-médullaire, comportant deux alésages (36, 38),dans la zone d'un tronçon d'accouplement proximal (21) se raccordant à sa tige de broche, traversant son axe longitudinal (A) et s'étendant par rapport à celui-ci sous un angle d'inclinaison (t), pour assurer le guidage d'une vis de traction ou d'une vis sans tête (26) pour assurer la compression de la hanche, **caractérisée en ce que**, pour une utilisation en tant que broche d'ostéosynthèse intra-médullaire (20) droite ou gauche, les embouchures des alésages sont ménagées avec un espacement périphérique (u) l'un par rapport à l'autre plus court que leur diamètre (i), les axes médians (M₁ ou M₂) des deux alésages délimitant, par un plan de référence (E) placés entre eux, passant chaque fois par l'axe longitudinal (A) de la broche d'ostéosynthèse intra-médullaire (20), chaque fois un angle de section transversale (n) d'environ 8°.

2. Broche d'ostéosynthèse intra-médullaire selon la revendication 1, **caractérisée en ce qu'**à chacun des deux alésages (36, 38) est associé, sous un espacement axial (h), un alésage (36ₐ, 38ₐ) parallèle pour une autre vis sans tête (26).

3. Broche d'ostéosynthèse intra-médullaire selon la revendication 1 ou 2, **caractérisée en ce que** les axes médians (M₁ ou M₂) des deux alésages (36 et 38) ou des paires d'alésages (36, 36ₐ et 38, 38ₐ) forment, observés en coupe transversale, un angle de 16°.

4. Broche d'ostéosynthèse intra-médullaire selon la revendication 2 ou 3, **caractérisée en ce qu'**un alésage (38ₐ ou 36) de l'autre paire d'alésages s'étend entre les alésages (36, 36ₐ ou 38, 38ₐ) d'une paire d'alésages parallèles.

5. Broche d'ostéosynthèse intra-médullaire selon l'une des revendications 1 à 4, **caractérisée en ce que** le plan de référence (E) est le plan de symétrie de la tige de broche (19) se raccordant au tronçon d'accouplement (21).

6. Broche d'ostéosynthèse intra-médullaire selon l'une des revendications 1 à 5, **caractérisée en ce que** le tronçon d'accouplement (21), dans la zone de son arête d'extrémité (42), présente au moins deux fentes d'engagement (40) à peu près parallèles à l'axe, ainsi qu'un filetage intérieur (44) pour un contre-filetage (70) d'un boulon adaptateur (60) devant assurer la liaison entre la broche d'ostéosynthèse intra-médullaire (20) et un étrier cible (46), une douille de guidage (48), traversée par le boulon adaptateur (60) d'un étrier cible (46), étant munie d'éléments conjugués (58) pour la fente d'engagement (40) de la broche d'ostéosynthèse intra-médullaire (20), qui coopèrent soit avec le (les) alésage(s) (36, 36ₐ) orientés à gauche, soit avec le (les) alésage(s) (38, 38ₐ) orientés à droite.

7. Broche d'ostéosynthèse intra-médullaire selon l'une des revendications 1 à 6, **caractérisée en ce que** la (les) vis sans tête (26) présente(nt) des cannelures longitudinales ainsi qu'un filetage auto-taraudeur.

8. Broche d'ostéosynthèse intra-médullaire avec des alésages transversaux pour des vis de verrouillage selon l'une des revendications 1 à 7, **caractérisée en ce que** la (les) vis de verrouillage (24) présente(nt) des cannelures longitudinales ainsi qu'un filetage auto-taraudeur.
